# EUROPEAN PATENT SPECIFICATION

(11) **EP 4 501 254 B1**
(45) Date of publication and mention of the grant of the patent: **18.03.2026**
(21) Application number: 23925587.0
(22) Date of filing: 12.04.2023
(51) Int. Cl.: A61B 17/221

(54) **MESH BASKET BODY AND CALCULUS REMOVING MESH BASKET**
NETZKORBKÖRPER UND ZAHNSTEIN ENTFERNENDER NETZKORB
CORPS DE PANIER MAILLÉ ET PANIER MAILLÉ DE RETRAIT DE CALCULS

(43) Date of publication of application: 05.02.2025
(73) Proprietor: Innovex Medical Co., Ltd., Shanghai 201201 (CN)
(72) Inventor: ZHENG, Zhongwei, Shanghai 201201 (CN); DING, Jiabo, Shanghai 201201 (CN); YAN, Hang, Shanghai 201201 (CN)
(74) Representative: Argyma
(86) International application number: PCT/CN2023/087855
(87) International publication number: WO 2024/212131

(56) References cited:
- WO-A1-2008/063713
- WO-A1-2015/175782
- WO-A1-98/48709
- CN-A- 105 873 528
- CN-A- 111 419 342
- CN-U- 213 883 392
- CN-U- 213 940 888
- CN-U- 213 940 888
- CN-U- 217 285 963
- US-A1- 2003 225 419
- US-A1- 2015 066 047

## Description

### TECHNICAL FIELD

The present invention relates to the technical field of medical instruments, and in particular to a basket body and a stone retrieval basket.

### BACKGROUND

In stone retrieval surgery, a large stone at a site is crushed by laser. Then the stone is retrieved by a stone retrieval basket under an endoscope. A surface of a conventional three-claw stone retrieval basket contacting a stone is made of polymer and metal materials. In normal use, an outer surface of the polymer material may be damaged by repeatedly rubbing with the hard stone, and the polymer material may be damaged at a contact surface between the polymer material and the metal material. A polymer tube has a smooth surface and a low friction coefficient, and the stone is easy to slip off.

WO2015/175782A1 and WO98/48709A1 disclose stone retrieval baskets having a plurality of sleeves connected by metal wires. The sleeves comprise a gripping surface.

### SUMMARY

In order to solve the above problems, the present invention provides a basket body according to claim 1.

Preferably, the anti-slip pattern penetrates through a tube wall of the metal sleeve.

Preferably, the outer surface of the metal sleeve is subjected to friction treatment.

Preferably, the friction treatment is performed by sand blasting or laser marking.

Preferably, a portion of the metal wire extending out of the metal sleeve is of a braided structure or a spring structure, or a braided structure or a spring structure is fixedly arranged on an outer surface of a portion of the metal wire extending out of the metal sleeve.

Preferably, a polymer conduit or a metal conduit is further fixedly arranged in each metal sleeve, and the metal wire is slidably inserted into the polymer conduit or the metal conduit.

Preferably, the metal sleeve is made of a stainless steel or nickel-titanium material, and the metal wire is a nickel-titanium wire.

The present invention further provides a stone retrieval basket, including the basket body mentioned above.

Compared with the prior art, the present invention has the following technical effect:
according to the basket body and the stone retrieval basket provided by the present invention, the sleeves of the basket body are made of the metal material, which enhances surface friction, and improves a grasping efficiency of the basket and the durability of repeated stone retrieval. Moreover, the anti-slip patterns are cut in the surfaces of the metal sleeves, which not only meets requirements on the flexibility of a head end of the basket, but also enhances the friction and durability of the basket and reduces the risk of a stone slipping from sides.

### BRIEF DESCRIPTION OF DRAWINGS

In order to explain the technical solutions in the embodiments of the present invention or in the prior art more clearly, the drawings used in the description of the embodiments or the prior art will be briefly introduced below. Clearly, the drawings in the following description are merely some embodiments of the present invention. For those of ordinary skill in the art, other drawings can be obtained based on these drawings without creative efforts.
FIG. 1 is a schematic diagram of a structure of a basket body in a closed state;
FIG. 2 is a schematic diagram of a structure of a basket body in an open state;
FIG. 3 and FIG. 4 are partial enlarged views of a basket body in an open state;
FIG. 5 is a schematic diagram of a structure of a metal sleeve having a sawtooth pattern cut in an outer surface;
FIG. 6 is a schematic diagram of a structure of a metal sleeve having a strip pattern cut in an outer surface;
FIG. 7 is a schematic diagram of a structure of a metal sleeve having a strip pattern and a spiral pattern cut in an outer surface according to an embodiment of the present invention;
FIG. 8 and FIG. 9 are schematic diagrams of a structure of a metal sleeve having a thread pattern cut in an outer surface;
FIG. 10 to FIG. 12 show a braided structure or a spring structure fixedly arranged on an outer surface of a portion of a metal wire extending out of a metal sleeve according to an embodiment of the present invention.

### DESCRIPTION OF EMBODIMENTS

The technical solutions in embodiments of the present invention will be clearly and fully described in combination with the drawings of the embodiments of the present invention; it is clear that the described embodiments are only a part of, and not all embodiments of, present invention. Based on the embodiments of the present invention, all other embodiments obtained by those of ordinary skill in the art without creative efforts should fall within the protection scope of the present invention.

The technical solutions of the present invention will be described in detail below by specific embodiments. The following several specific embodiments may be mutually combined, and same or similar concepts or processes may not be repeatedly described in some embodiments.

### Embodiment 1

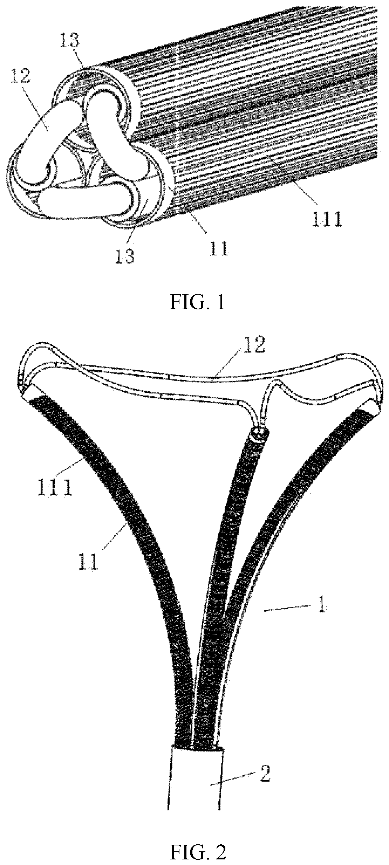

Refer to FIG. 1 to FIG. 9, a basket body 1 includes a plurality of metal sleeves 11 arranged in parallel, each metal sleeve 11 being of a tubular structure with two open ends. The present invention does not limit what metal material the metal sleeve 11 is made of, for example stainless steel or high elastic material such as nickel titanium, with an objective of firmly grasping a foreign object such as a stone by a medical hand, without easily slipping off. Furthermore, anti-slip patterns 111 are cut in outer surfaces of the metal sleeves 11, which aims to improve a friction coefficient of the metal sleeves 11, preventing the foreign object from slipping off while grasping the foreign object, such that the metal sleeves 11 are easier to grasp the foreign object, and surgical time is saved.

Each two adjacent metal sleeves 11 are connected by a metal wire 12: a polymer conduit (the polymer conduit is made of a polymer material) or a metal conduit 13 is fixedly arranged in each metal sleeve 11, each metal wire 12 is slidably inserted into the corresponding polymer conduit or metal conduit 13, that is, the metal wire 12 can slide relative to the polymer conduit or metal conduit 13 where the metal wire is located, and the polymer conduit or metal conduit 13 can ensure that the metal wire can slide freely therein. A front end of each metal wire 12 extends out of a front end opening of the corresponding metal sleeve 11 (the front end refers to an end far away from an operator during surgery) , and extends into a front end opening of the adjacent metal sleeve 11 for fixation, that is, one end of the metal wire 12 is a fixed end, and the fixed end is fixedly connected to the corresponding metal sleeve 11, for example, the fixed end is fixed into the corresponding metal sleeve 11 by bonding or welding. The other end of the metal wire 12 is a free end, which can slide in the polymer conduit or metal conduit 13 in the adjacent metal sleeve 11. When the free ends of the metal wires 12 extend forward along the polymer conduits or metal conduits 13, the basket body 1 is opened, and the metal sleeves 11 can clamp a foreign object such as a stone in a gap after slight transformation, and the foreign object is not easy to fall off. When the free ends of the metal wires 12 are withdrawn backward along the polymer conduits or metal conduits 13, the basket body 1 is closed. The anti-slip patterns 111 are cut in the surfaces of the metal sleeves 11, such that the surface friction coefficient is large, and the foreign object is not easy to fall off.

The metal sleeves 11 in this embodiment are made of a metal material, because the metal material is more durable than the polymer material, and more fatigue-resistant than a polymer tube. Furthermore, during repeated opening and closing of the basket, the foreign object rubs continuously, which may damage the polymer tube. The metal sleeves 11 can overcome this shortcoming, that is, the friction coefficient of the surfaces of the metal sleeves 11 is enhanced, and a grasping efficiency of the basket and the durability of repeated stone retrieval are improved. Moreover, the anti-slip patterns 111 are cut in the surfaces of the metal sleeves 11, which not only meets requirements on the flexibility of a head end of the basket, but also enhances the friction and durability of the basket and reduces the risk of a foreign object such as a stone slipping from sides.

When the basket body is opened, the metal sleeves 11 elastically bend outward, such that with the anti-slip patterns 111 cut in the metal sleeves 11, on one hand, the metal sleeves 11 generate elastic deformation under the action of an external force, that is, the elasticity of the metal sleeves 11 is improved, and on the other hand, the friction force of the outer surfaces of the metal sleeves 11 is increased, that is, the friction coefficient of the outer surfaces of the metal sleeves 11 is improved.

Refer to FIG. 2, in an open state, each metal wire 12 extends forward from the polymer conduit or metal conduit 13 where the metal wire is located, and meanwhile, the metal sleeves 11 expand outward, and the plurality of metal sleeves 11 and the metal wires 12 are combined to form a conical basket.

The present invention does not limit the numbers of the metal sleeves 11 and the metal wires 12, and the numbers of the metal sleeves 11 and the metal wires 12 are the same. For example, the numbers of the metal sleeves 11 and the metal wires 12 may be three or more separately, and the numbers of the metal sleeves 11 and the metal wires 12 are preferably three separately in this embodiment.

In the present invention, the anti-slip patterns 111 are formed by cutting, and the anti-slip patterns 111 may be hollow patterns (that is, the anti-slip patterns 111 penetrate through tube walls of the metal sleeves 11), and may not be hollow patterns (that is, the anti-slip patterns 111 do not penetrate through the tube walls of the metal sleeves 11), which is not limited in the present invention. The following is a detailed description of several specific anti-slip patterns 111.

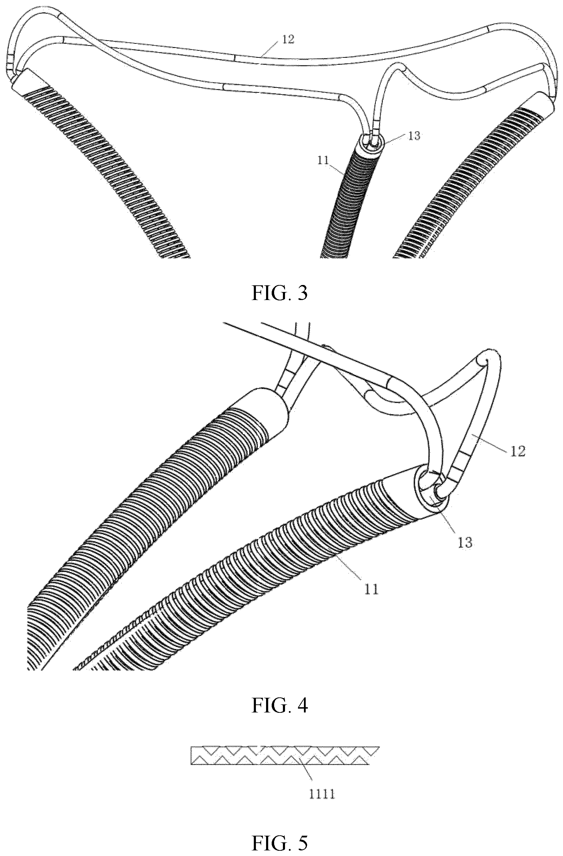

Refer to FIG. 5, as a first example, the anti-slip pattern 111 is a sawtooth pattern.

The sawtooth pattern is formed by a plurality of sawtooth slots 1111 cut at intervals in the outer surface of the metal sleeve 11 in an axial direction, that is, a plurality of sawtooth slots 1111 are cut in the outer surface of the metal sleeve 11 in an axial direction. A slot width of the sawtooth slots 1111 is not specifically limited in this example, and can be set according to the size of the stone. If the stone is large, the slot width of the sawtooth slots 1111 is large. If the stone is small, the slot with of the sawtooth slots 1111 is small. The sawtooth pattern provided is more suitable for a large-scale stone. The metal sleeves 11 are not easy to be extruded and deformed again in a longitudinal direction with the grasping of the sawtooth slots.

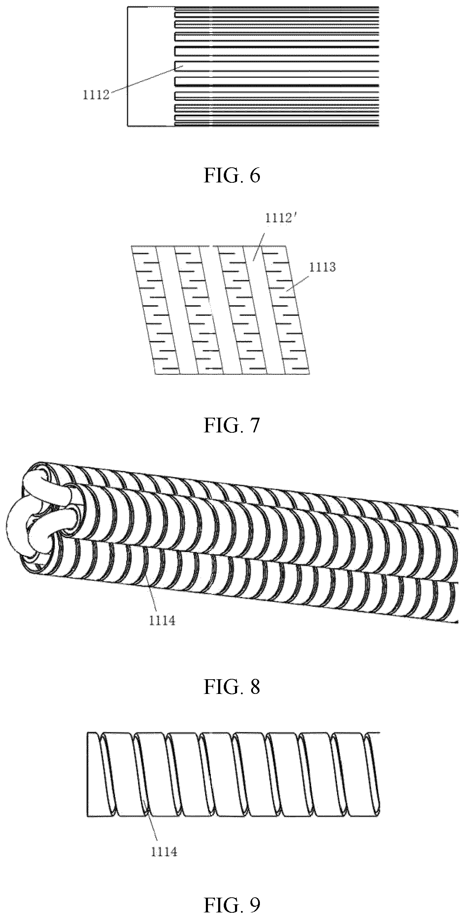

Refer to FIG. 6, as a second example, the anti-slip pattern 111 is a strip pattern. The strip pattern is formed by a plurality of strip slots 1112 cut at intervals in the outer surface of the metal sleeve 11 in an axial direction, that is, a plurality of strip slots 1112 are cut in the outer surface of the metal sleeves 11 in an axial direction. The strip pattern is more suitable for grasping a small-scale stone, preferably, the slot width of the strip slots 1112 is less than a diameter of the metal wire 12.

Refer to FIG. 7, as an embodiment of the present invention, the anti-slip pattern 111 includes a strip pattern and a spiral pattern. The strip pattern is formed by a plurality of strip slots 1112' cut at intervals in the outer surface of the metal sleeve 11 in an axial direction, that is, a plurality of strip slots 1112' are cut in the outer surface of the metal sleeve 11 in an axial direction. The spiral pattern is formed by spiral slots 1113 cut in the outer surface of the metal sleeve 11 in a longitudinal direction between each two adjacent strip slots 1112', that is, the spiral slots 1113 are cut in the outer surface of the metal sleeve 11 in a longitudinal direction between each two adjacent strip slots 1112'. The anti-slip pattern 111 can be suitable for grasping the large-scale stone, and can also be suitable for grasping the small-scale stone.

Refer to FIG. 8, as a third example, the anti-slip pattern 111 is a thread pattern. The thread pattern is formed by threads 1114 spirally cut in the outer surface of the metal sleeve 11 in a longitudinal direction, that is, the threads 1114 are spirally cut in the outer surface of the metal sleeve 11 in a longitudinal direction. The smaller the pitch of the threads 1114, the softer the metal sleeve 11 and the easier the deformation of the basket. The thread pattern is suitable for grasping the small-scale stone. In this example, slopes of the threads of the anti-slip pattern 111 may be the same or different, When the slopes of the threads of the anti-slip pattern 111 are different, by adjusting the slopes of the threads, the whole softness of the metal sleeve 11can be adjusted, and basket products can be made for meeting different use scenarios, for example, the metal sleeve 11 can have a soft front end and a hard rear end (that is, the slope of the front end is less than the slope of the rear end), or have a hard front end and a soft rear end (that is, the slope of the front end is greater than the slope of the rear end).

In order to further increase the friction coefficient of the outer surface of the metal sleeve 11, the outer surface of the metal sleeve 11 is subjected to friction treatment, for example, the outer surface of the metal sleeve 11 is processed by sand blasting or laser marking to enhance friction, which not only meets requirements on the flexibility of a head end of the basket, but also enhances the friction and durability of the basket and reduces the risk of the stone slipping from sides.

In this embodiment, the metal wire 12 belongs to a mature technology in the field, the material of which is not limited in the present invention, and the metal wire 12 in the field is a nickel-titanium wire. Certainly, the metal wire may also be made of other high elastic metal materials, which is not specifically limited the present invention.

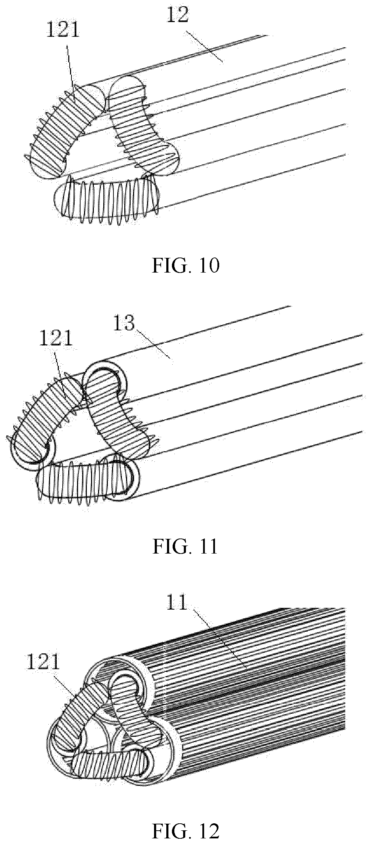

In order to further increase the friction of the head of the basket, and reduce the risk of the stone falling from the top end, all or part of the outer surface of the metal wire 12 may be subjected to friction treatment. Refer to FIG. 10 to FIG. 12, in order not to destroy the overall strength of the metal wire 12 and reduce the costs, in this embodiment, preferably, a portion of the metal wire 12 extending out of the metal sleeve 11 is of a braided structure or a spring structure, or a braided structure or a spring structure 121 is fixed on an outer surface of a portion of the metal wire 12 extending out of the metal sleeve 11, so as to increase the friction of the metal wire 12. The braided structure or the spring structure 121 may be a portion extending out of the metal sleeve 11 when the basket body 1 is in a closed state, or may be a portion extending out of the metal sleeve 11 when the basket body 1 is in an open state. In order to prevent the braided structure or the spring structure 121 from being stuck in the polymer conduit or the metal conduit 13 with the opening of the basket body 1, and/or being unable to be retracted into the polymer conduit or the metal conduit 13 with the closing of the basket body 1, in this embodiment, preferably, the portion extending out of the metal sleeve 11 is provided with the braided structure or the spring structure when the basket body 1 is in the closed state.

### Embodiment 2

Refer to FIG. 2, this embodiment provides a stone retrieval basket, including the basket body 1 according to the above embodiment, and a sheath 2. Rear ends of a plurality of metal sleeves 11 of the basket body 1 (the rear end refers to an end close to an operator during surgery) are in communication with the sheath 2, and are fixedly connected to a front end of the sheath 2.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments without making the essence of the corresponding technical solutions departing from the scope of the technical solutions of the embodiments of the present invention.

## Claims

1. A basket body (1), comprising a plurality of metal sleeves (11) arranged in parallel, an anti-slip pattern (111) being cut in an outer surface of each metal sleeve (11); each two adjacent metal sleeves (11) are connected by a metal wire (12), the metal wire (12) is slidably inserted into one metal sleeve (11), and a front end of the metal wire (12) extends out of a front end opening of the metal sleeve (11), and extends into a front end opening of an adjacent metal sleeve (11) for fixation; and
in an open state, the plurality of metal sleeves (11) and the metal wires (12) are combined to form a conical basket, the anti-slip pattern (111) comprises a strip pattern formed by a plurality of strip slots (1112') cut at intervals in the outer surface of the metal sleeve (11) in an axial direction and a spiral pattern formed by spiral slots (1113) cut in the outer surface of the metal sleeve (11) in a longitudinal direction between each two adjacent strip slots (1112').

2. The basket body (1) according to claim 1, wherein the anti-slip pattern (111) penetrates through a tube wall of the metal sleeve (11).

3. The basket body (1) according to claim 1, wherein the outer surface of the metal sleeve (11) is subjected to friction treatment.

4. The basket body (1) according to claim 3, wherein the friction treatment is performed by sand blasting or laser marking.

5. The basket body (1) according to claim 1, wherein a portion of the metal wire (12) extending out of the metal sleeve (11) is of a braided structure or a spring structure (121) , or a braided structure or a spring structure (121) is fixedly arranged on an outer surface of a portion of the metal wire (12) extending out of the metal sleeve (11).

6. The basket body (1) according to claim 1, wherein a polymer conduit or a metal conduit (13) is further fixedly arranged in each metal sleeve (11), and the metal wire (12) is slidably inserted into the polymer conduit or the metal conduit (13).

7. The basket body (1) according to claim 1, wherein the metal sleeve (11) is made of a stainless steel or nickel-titanium material, and the metal wire (12) is a nickel-titanium wire.

8. A stone retrieval basket, comprising the basket body (1) according to any one of claims 1 to 7.

## Patentansprüche

1. Korbkörper (1), der eine Mehrzahl von Metallhülsen (11) umfasst, die parallel angeordnet sind, wobei ein Antirutschmuster (111) in eine Außenfläche jeder Metallhülse (11) geschnitten ist; jeweils zwei benachbarte Metallhülsen (11) durch einen Metalldraht (12) verbunden sind, der Metalldraht (12) gleitfähig in eine Metallhülse (11) eingefügt ist und sich ein Vorderende des Metalldrahts (12) aus einer Vorderendöffnung der Metallhülse (11) erstreckt und sich zur Fixierung in eine Vorderendöffnung einer benachbarten Metallhülse (11) erstreckt; und
in einem offenen Zustand die Mehrzahl von Metallhülsen (11) und die Metalldrähte (12) so kombiniert sind, dass sie einen kegelförmigen Korb bilden, das Antirutschmuster (111) ein Streifenmuster, das von einer Mehrzahl von Streifenschlitzen (1112') gebildet ist, die in einer Axialrichtung in Abständen in die Außenfläche der Metallhülse (11) geschnitten sind, und ein Spiralmuster, das von Spiralschlitzen (1113) gebildet ist, die in einer Längsrichtung zwischen jeweils zwei benachbarten Streifenschlitzen (1112') in die Außenfläche der Metallhülse (11) geschnitten sind, umfasst.

2. Korbkörper (1) nach Anspruch 1, wobei das Antirutschmuster (111) eine Röhrenwand der Metallhülse (11) durchdringt.

3. Korbkörper (1) nach Anspruch 1, wobei die Außenfläche der Metallhülse (11) einer Reibungsbehandlung unterzogen ist.

4. Korbkörper (1) nach Anspruch 3, wobei die Reibungsbehandlung durch Sandstrahlen oder Lasermarkieren durchgeführt wird.

5. Korbkörper (1) nach Anspruch 1, wobei ein Abschnitt des Metalldrahts (12), der sich aus der Metallhülse (11) erstreckt, eine Flechtstruktur oder eine Federstruktur (121) aufweist, oder eine Flechtstruktur oder eine Federstruktur (121) fest an einer Außenfläche eines Abschnitts des Metalldrahts (12) angeordnet ist, der sich aus der Metallhülse (11) erstreckt.

6. Korbkörper (1) nach Anspruch 1, wobei eine Polymerleitung oder eine Metallleitung (13) ferner fest in jeder Metallhülse (11) angeordnet ist und der Metalldraht (12) gleitfähig in die Polymerleitung oder die Metallleitung (13) eingefügt ist.

7. Korbkörper (1) nach Anspruch 1, wobei die Metallhülse (11) aus einem Edelstahl- oder Nickel-Titanmaterial hergestellt und der Metalldraht (12) ein Nickel-Titandraht ist.

8. Steinentnahmekorb umfassend einen Korbkörper (1) nach einem der Ansprüche 1 bis 7.

## Revendications

1. Un corps de panier (1), comprenant une pluralité de manchons métalliques (11) disposés en parallèle, un motif antidérapant (111) étant découpé dans une surface externe de chaque manchon métallique (11) ; chaque paire de manchons métalliques adjacents (11) sont reliés par un fil métallique (12), le fil métallique (12) est inséré de manière coulissante dans un manchon métallique (11), et une extrémité avant du fil métallique (12) s'étend hors d'une ouverture d'extrémité avant du manchon métallique (11), et s'étend à l'intérieur d'une ouverture d'extrémité avant d'un manchon métallique adjacent (11) pour la fixation ; et
dans un état ouvert, la pluralité de manchons métalliques (11) et les fils métalliques (12) sont combinés pour former un panier conique, le motif antidérapant (111) comprend un motif de bande formé par une pluralité de fentes en bande (1112') découpées à des intervalles dans la surface externe du manchon métallique (11) dans une direction axiale et un motif en spirale formé par des fentes en spirale (1113) découpées dans la surface externe du manchon métallique (11) dans une direction longitudinale entre chaque paire de fentes en bande adjacentes (1112').

2. Le corps de panier (1) selon la revendication 1, dans lequel le motif antidérapant (111) pénètre à travers une paroi tubulaire du manchon métallique (11).

3. Le corps de panier (1) selon la revendication 1, dans lequel la surface externe du manchon métallique (11) est soumise à un traitement par frottement.

4. Le corps de panier (1) selon la revendication 3, dans lequel le traitement par frottement est exécuté par sablage ou marquage au laser.

5. Le corps de panier (1) selon la revendication 1, dans lequel une partie du fil métallique (12) s'étendant hors du manchon métallique (11) est constituée d'une structure tressée ou d'une structure à ressort (121), ou une structure tressée ou une structure à ressort (121) est disposée fixement sur une surface externe d'une partie du fil métallique (12) s'étendant hors du manchon métallique (11).

6. Le corps de panier (1) selon la revendication 1, dans lequel un conduit polymère ou un conduit métallique (13) est en outre disposé fixement dans chaque manchon métallique (11), et le fil métallique (12) est inséré de manière coulissante dans le conduit polymère ou le conduit métallique (13).

7. Le corps de panier (1) selon la revendication 1, dans lequel le manchon métallique (11) est constitué d'un matériau en acier inoxydable ou en nickel-titane, et le fil métallique (12) est un fil en nickel-titane.

8. Un panier de récupération de pierres, comprenant le corps de panier (1) selon l'une quelconque des revendications 1 à 7.
